# EUROPEAN PATENT SPECIFICATION

(11) **EP 0 936 211 B1**
(45) Date of publication and mention of the grant of the patent: **07.07.2010**
(21) Application number: 99810036.6
(22) Date of filing: 19.01.1999
(51) Int. Cl.: C07C 69/732, C07C 229/44, C11D 3/50, A61K 8/00

(54) **Aryl-acrylic acid esters useful as precursors for organoleptic compounds**
Arylacrylsäureester, verwendbar als Vorstufen von organoleptischen Verbindungen
Esters de l'acide arylacrylique, utilisés en tant que précurseurs de composés organoleptiques

(30) Priority: 13.02.1998 EP 98810114
(43) Date of publication of application: 18.08.1999
(73) Proprietor: Givaudan SA, 1214 Vernier-Genève (CH)
(72) Inventor: Anderson, Denise, Dr., 8032 Zürich (CH); Fráter, Georg, Dr., 8400 Winterthur (CH)
(74) Representative: Givaudan Patents

(56) References cited:
- EP-A- 0 816 322
- EP-A- 0 887 335
- EP-A- 0 887 336
- WO-A-97/30687
- KIRKHAM D S ET AL : "Studies of the in vivo activity of esters of o-coumaric and cinnamic acids against apple scab" ANN. APPL. BIOL., vol. 55, 1965, pages 359-371, XP002104194
- SNOOK M E ET AL: "Characterization and Quantitation of Hexadecyl, Octadecyl, and Eicosyl Esters of p-Coumaric Acid in the Vine and Root Latex of Sweetpotato" J. AGRIC. FOOD CHEM., vol. 42, no. 11, 1994, pages 2589-2595, XP002104195
- TOYOTA M ET AL: "Sesquiterpenes from Japanese Liverworts" PHYTOCHEMISTRY, vol. 20, no. 10, 1981, pages 2359-2366, XP002104196
- MCCLUSKEY K L ET AL: "Menthol Studies. I. Menthyl Esters of the Nitro- and Aminocinnamic acids" J. AM. CHEM. SOC. , vol. 49, no. 1, January 1927, pages 452-457, XP002104197
- HEMMERLE H ET AL: "Chlorogenic Acid and Synthetic Chlorogenic Acid Derivatives: Novel Inhibitors of Hepatic Glucose-6-phosphate Translocase" J. MED. CHEM., vol. 40, no. 2, 1997, pages 137-145, XP002078904
- DATABASE XFIRE Beilstein Informationssysteme GmbH, Frankfurt, DE XP002104202 & NICHIFORESCO E ET AL: ANN. PHARM. FR., vol. 23, no. 6, 1965, pages 419-427,
- LUONG M D ET AL: "Contribution à la phytochimie du genre Gentiana. XXVI. Identification d'une nouvelle di-O-glucosyl cinnamoyl-C-glucosylflavone extraite des feuilles de Gentiana X marcailhouana Ry" HELV. CHIM. ACTA , vol. 64, no. 8, 1981, pages 2741-2745, XP002104199
- GALABOV A S ET AL : "Antiviral Activity of Cholersteryl Esters of Cinnamic Acid Derivatives" Z. NATURFORSCH., vol. 53c, 1998, pages 883-887, XP002104200
- DEY B B ET AL: "Geometrical Inversion in the Acids Derived from the Coumarins" J. INDIAN CHEM., vol. 11, 1934, pages 743-749, XP002104201

## Description

The present invention relates to 3-(2-substituted aryl)-acrylic acid esters, especially 3-(2-hydroxyaryl)-acrylic acid esters and 3-(2-aminoaryl)-acrylic acid esters. These compounds are useful as precursors for organoleptic compounds, especially for flavours, fragrances and masking agents, antimicrobial compounds and for fluorescent whitening agents.

A principal strategy currently employed in imparting odours to consumer products is the admixing of the fragrance directly into the product. There are, however, several drawbacks to this strategy. The fragrance material can be too volatile and/or too soluble, resulting in fragrance loss during manufacturing, storage, and use. Many fragrance materials are also unstable over time. This again results in loss during storage.

In many consumer products it is desirable for the fragrance to be released slowly over time. Microencapsulation and inclusion complexes with cyclodextrins have been used to help decrease volatility, improve stability and provide slow-release properties. However, these methods are for a number of reasons often not successful. In addition, cyclodextrins can be too expensive.

Fragrance precursors for scenting fabrics being washed in the presence of a lipase-containing detergent are described in WO 95/04809. The fragrance precursors contained in the detergent and/or in the softener are cleaved by the lipase and a single active compound, either an odoriferous alcohol or aldehyde or ketone is yielded. Thereby a prolonged scenting effect on the fabric is obtained. The need for a lipase-containing detergent is limiting. In many parts of the world, detergents do not contain lipase. Other consumers prefer to use 'nonbio' detergents.

Fragrance precursors generating in the presence of skin bacteria or enzymes, acidic or alkaline conditions organoleptic compounds are described in EP 0 816 322, EP 0 887 335 and EP 0 887 336.

WO97/30687 refers to fragrance precursors which upon contact with the skin produce fragrances.

J. Indian Chem. 11, 743-749 (1934) describes the conversion of o-coumaric acid, for example methyl coumarate and ethylcoumarate, and its nitro and methoxy derivatives into coumarines.

Fluorescent whitening agents or brighteners have been added to laundry detergents since the 1950s to help maintain the original brightness of white clothing.

One object of the present invention is to provide new precursors for compounds with different activities and thus impart different activities to a product by the addition of just one compound. A further object of the invention is to provide new compounds which are stable under transport and storage conditions. A further object of the present invention is to provide precursor molecules supplying different active compounds simultaneously or successively.

The present invention relates to 3-(2-substituted aryl)-acrylic acid esters of the formula I 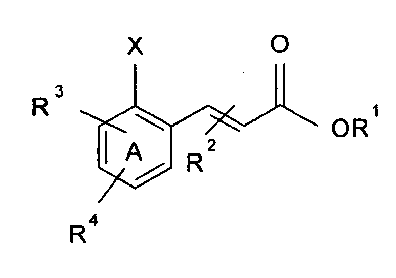 wherein
A is a benzene,
R¹ is a saturated or unsaturated, straight, branched, alicyclic or aromatic C₁₀-C₃₀ hydrocarbon residue, or
R¹ is a saturated or unsaturated, straight, branched, alicyclic or aromatic C₁₀-C₃₀ hydrocarbon residue containing one or more O and /or N atoms and/or C(O) groups and/or alkoxy groups, or
R¹ is a saturated or unsaturated, straight, branched, alicyclic or aromatic C₁₀-C₃₀ hydrocarbon residue substituted by an ionic substituent of formula N(R⁵)₃⁺, in which R⁵ is the residue of an alkyl group with 1 to 30 carbon atoms;
R² in 2- or 3-position is methyl, or
R² in 2- or 3-position is a substituted heterocyclic residue of the formula 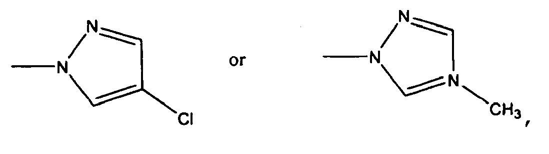
R³ and R⁴ stand for hydrogen, a straight or branched C₁-C₆ alkyl or C₁-C₆ alkoxy residue,a five membered heterocyclic residue containing N and/or O atoms, a five membered heterocyclic residue substituted by C₁-C₆ aliphatic and/or aromatic substituents, or -OH,
R², R³ and R⁴ may be the same or different,
X stands for -OH or NHR⁶, wherein R⁶ is hydrogen a saturated or unsaturated, straight or branched C₁-C₂₀ hydrocarbon or an aromatic or heterocyclic residue,
and the acrylic double bond is of the E configuration.

In the above formula I, all possible enantiomers and diastereomers and all mixtures are included within the scope of the invention.

Compounds wherein R¹ is a saturated or unsaturated, straight or branched C₁₀-C₃₀ hydrocarbon residue containing one or more O and/or N atoms and/or C(O) groups and/or alkoxy groups or substituted by an ionic substituent of the formula (NR⁵)₃⁺, in which R⁵ is the residue of a fatty acid or an alkyl group with 1 to 30 carbon atoms are preferred.

R² is preferably a heterocyclic residue of the formula

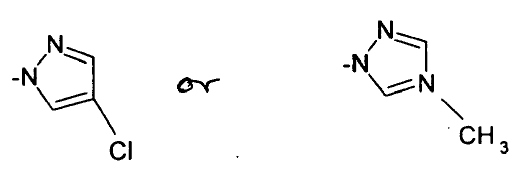

R³ and/or R⁴ are preferably hydrogen, a five membered heterocyclic residue optionally containing N and/or O atoms, substituted by C₁-C₆ aliphatic and/or aromatic substituents.

R¹ may be the residue of an olfactory alcohol of the formula R¹OH, the enol form of an olfactory aldehyde of the formula R¹HO or of an olfactory ketone of formula R¹O. R¹ may also be an optionally substituted alkyl, alkenyl or arylalkyl residue carrying an 1-alkoxy, 1-aryloxy, or 1-arylalkoxy residue.

The compounds of formula I are mostly or nearly odourless at room temperature, atmospheric conditions and about 20 to 100 % relative humidity. However, under activating conditions, they are cleaved. Thereby the residue of formula Ia 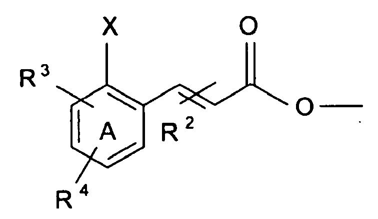 yields a coumarin of the formula II 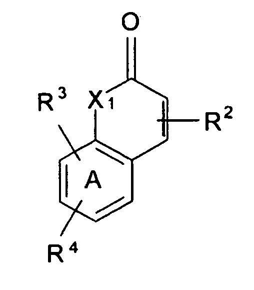 wherein X¹ stands for O or NR⁶, R²,R³, R⁴ have the meaning as defined above. The coumarins of formula II can have organoleptic and/or antimicrobial properties and/or optical brightening activity. Preferred are coumarins with olfactory properties. If R¹ is the residue of an olfactory alcohol or of the enol form of an olfactory aldehyde or ketone, upon cleavage two active compounds with the above properties can be obtained. Thus, the compounds of formula I permit the development of useful consumer products with enhanced organoleptic and/or antimicrobial properties and/or optical brightening properties. The organoleptic coumarins and alcohols or aldehydes or ketones obtained are useful as fragrances, flavours, masking agents and antimicrobial agents.

The activating conditions which lead to cleavage and thereby to the desired active compounds comprise the presence of UV light such as sunlight and elevated temperatures.

The invention relates also to the use of compounds of formula I 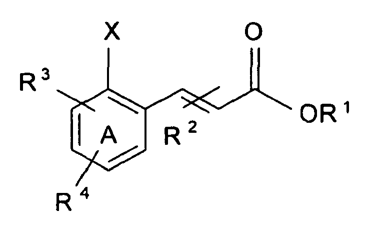 wherein
A is a benzene,
R¹ is hydrogen, unsaturated or saturated, straight or branched, alicyclic or aromatic C₁₀-C₃₀ hydrocarbon residue, or
R¹ is a saturated or unsaturated, straight, branched, alicyclic or aromatic C₁₀-C₃₀ hydrocarbon residue containing one or more O and /or N atoms and/or C(O) groups and/or alkoxy groups, or
R¹ is a saturated or unsaturated, straight, branched, alicyclic or aromatic C₁₀-C₃₀ hydrocarbon residue substituted by an ionic substituent of formula N(R⁵)₃⁺, in which R⁵ is the residue of an alkyl group with 1 to 30 carbon atoms;
R² in 2- or 3-position is methyl, or
R² in 2- or 3-position is a substituted heterocyclic residue of the formula 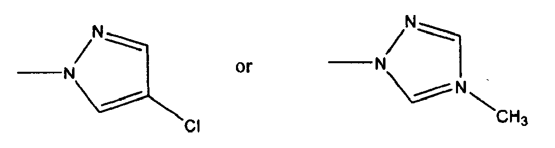
R³ and R⁴ stand for hydrogen, a straight or branched C1-C6 alkyl or C1-C6 alkoxy residue, a five membered heterocyclic residue containing N and/or O atoms, a five membered heterocyclic residue substituted by C₁-C₆ aliphatic and/or aromatic substituents, or -OH,
R², R³ and R⁴ may be the same or different,
X stands for -OH or NHR⁶, wherein R⁶ is hydrogen a saturated or unsaturated, straight or branched C₁-C₂₀ hydrocarbon or an aromatic or heterocyclic residue,
and the acrylic double bond is of the E configuration,
including such compounds of formula I wherein R² is hydrogen and such compounds wherein R¹ is the residue of an alcohol R¹OH selected from the group consisting of amyl alcohol, hexyl alcohol, 2-hexyl alcohol, heptyl alcohol, octyl alcohol, nonyl alcohol, 3-methyl-but-2-en-1-ol, 3-methyl-1-pentanol, cis-3-hexenol, cis-4-hexenol, 3,5,5-trimethyl-hexanol, oct-1-en-3-ol, cis-6-nonenol, 2,6-nonadien-1-ol, benzylalcohol, 1-phenyl-ethanol, 2-phenyl-ethanol, 2-phenyl-propanol, 3-phenyl-propanol, 2-(2-methylphenyl)-ethanol, 2-phenoxy-ethanol, 2-methoxy-4-methyl-phenol, 4-methyl-phenol, anisic alcohol, p-tolyl alcohol, cinnamic alcohol, vanillin, ethyl vanillin, 4-isopropyl-cyclohexanol, 3,3,5-trimethyl-cyclohexanol, 2-cyclohexyl-propanol and 2,6-dimethyl-heptan-2-ol; or R¹ is the residue of the enol form of an aldehyde R¹HO selected from the group consisting of 2,4-dimethyl-cyclohex-3-ene-1-carboxaldehyde, 1-carboxaldehyde-2,4-dimethyl-cyclohex-3-ene, 3,5,5-trimethyl-hexanal, heptanal, octanal, nonanal, 3,5,5-trimethylhexanal, 2-phenyl-propanal, 4-methyl-phenyl-acetaldehyde and 2,3,5,5,-tetramethyl-hexanal;
as precursors for organoleptic compounds, e.g. flavours, fragrances, odour masking agents and as precursors for antimicrobial agents and as precursors for fluorescent whitening agents.

Preferred substituents are mentioned above.

The esters of formula I can act as fragrance precursors in laundry products. They can also act as precursors for odour masking agents in the same products as the fragrance precursors. They also can act as precursors for antimicrobial agents. In addition, they can also act as precursors for fluorescent whitening agents. The fragrance precursors and the precursors for odour masking agents as well as the flavour precursors of the invention may be used individually in an amount effective to enhance or to mask the characteristic odour or flavour of a material. More commonly, however, the compounds are mixed with other fragrance or flavour components in an amount sufficient to provide the desired odour or flavour characteristics.

The brightener precursors may be used also individually in an effective amount and mixed with one or more other brightener or colorant substance.

Due to the in situ generation of the active compounds the desired effect is prolonged and the substantivity on different substrates is enhanced. If two active compounds are provided by one ester of the formula I, they can be generated, depending on the precursor and/or the activating conditions, simultaneously or successively. Further, the precursors of the invention provide slow release of the active compounds.

Examples of alcohols R¹OH generated upon cleavage and constituting the residue R¹ in the compounds of formula I are:
amyl alcohol
hexyl alcohol*
2-hexyl alcohol*
heptyl alcohol*
octyl alcohol*
nonyl alcohol*
decyl alcohol*
undecyl alcohol*
lauryl alcohol*
myristic alcohol
3-methyl-but-2-en-1-ol*
3-methyl-1-pentanol
cis-3-hexenol*
cis-4-hexenol*
3,5,5-trimethyl-hexanol
3,4,5,6,6-pentamethylheptan-2-ol*
citronellol*
geraniol*
oct-1-en-3-ol
2,5,7-trimethyl-octan-3-ol
2-cis-3,7-dimethyl-2,6-octadien-1-ol
6-ethyl-3-methyl-5-octen-1-ol*
3,7-dimethyl-oct-3,6-dienol*
3,7-dimethyloctanol*
7-methoxy-3,7-dimethyl-octan-2-ol*
cis-6-nonenol*
5-ethyl-2-nonanol
6,8-dimethyl-2-nonanol*
2,2,8-trimethyl-7(8)-nonene-3-ol
nona-2,6-dien-1-ol
4-methyl-3-decen-5-ol*
dec-9-en-1-ol
benzylalcohol
2-methyl-undecanol
10-undecen-1-ol
1-phenyl-ethanol*
2-phenyl-ethanol*
2-methyl-3-phenyl-3-propenol
2-phenyl-propanol*
3-phenyl-propanol*
4-phenyl-2-butanol
2-methyl-5-phenyl-pentanol*
2-methyl-4-phenyl-pentanol*
3-methyl-5-phenyl-pentanol*
2-(2-methylphenyl)-ethanol*
4-(1-methylethyl)-benzene-methanol
4-(4-hydroxyphenyl)-butan-2-one*
2-phenoxy-ethanol*
4-(1-methylethyl)-2-hydroxy-1-methyl benzene
2-methoxy-4-methyl-phenol
4-methyl-phenol
anisic alcohol*
p-tolyl alcohol*
cinnamic alcohol*
vanillin*
ethyl vanillin*
eugenol*
isoeugenol*
thymol
anethol*
decahydro-2-naphthalenol
borneol*
cedrenol*
farnesol*
fenchyl alcohol*
menthol*
3,7,11-trimethyl-2,6,10-dodecatrien-1-ol
alpha ionol*
tetrahydro ionol*
2-(1,1-dimethylethyl)cyclohexanol*
3-(1,1-dimethylethyl)cyclohexanol*
4-(1,1-dimethylethyl)cyclohexanol*
4-isopropyl-cyclohexanol
6,6-dimethyl-bicyclo[3.3.1]hept-2-ene-2-ethanol
6,6-dimethyl-bicyclo[3.1.1]hept-2-ene-methanol*
p-menth-8-en-3-ol*
3,3,5-trimethyl-cyclohexanol
2,4,6-trimethyl-3-cyclohexenyl-methanol*
4-(1-methylethyl)-cyclohexyl-methanol*
4-(1,1-dimethylethyl)-cyclohexanol
2-(1,1-dimethylethyl)-cyclohexanol
2,2,6-trimethyl-alpha-propyl-cyclohexane propanol*
5-(2,2,3-trimethyl-3-cyclopentenyl)-3-methylpentan-2-ol*
3-methyl-5-(2,2,3-trimethylcyclopentyl-3-enyl)pent-4-en-2-ol*
2-ethyl-4(2,2,3-trimethylcyclopentyl-3-enyl)but-2-en-1-ol*
4-(5,5,6-trimethylbicyclo[2.2.1]hept-2-yl)-cyclohexanol*
2-(2-methylpropyl)-4-hydroxy-4-methyl-tetrahydropyran*
2-cyclohexyl-propanol*
2-(1,1-dimethylethyl)-4-methyl-cyclohexanol*
1-(2-tert-butyl-cyclohexyloxy)-2-butanol*
1-(4-isopropyl-cyclohexyl)-ethanol*
2,6-dimethyl-oct-7-en-2-ol**
2,6-dimethyl-heptan-2-ol**
3,7-dimethyl-octa-1,6-dien-3-ol**
whereby * indicates the preferred alcohols and ** indicate the more preferred alcohols.

Examples of aldehydes R¹HO generated upon cleavage and constituting the residue R¹ in the compounds of formula I are
2,6,10-trimethylundec-9-enal*
1,2,3,4,5,6,7,8,-octahydro-8,8-dimethyl-2-napthalenecarboxaldehyde
tridecanal
2-[4-(1-methylethyl)phenyl]-ethanal
2,4-dimethyl-cyclohex-3-ene-1-carbox-aldehyde*
4-carboxaldehyde-1,3,5-trimethyl-cyclohex-1-ene*
1-carboxaldehyde-2,4-dimethyl-cyclohex-3-ene*
1-carboxaldehyde-4-(4-hydroxy-4-methylpentyl)-cyclohex-3-ene*
3,5,5-trimethyl-hexanal
heptanal*
2,6-dimethyl-hept-5-enal*
decanal**
dec-9-enal
dec-4-enal
2-methyldecanal*
undec-10-enal**
undecanal*
dodecanal**
2-methyl-undecanal**
tridecanal
octanal**
nonanal*
3,5,5-trimethylhexanal
undec-9-enal**
2-phenyl-propanal*
4-methyl-phenyl-acetaldehyde*
3,7-dimethyl-octanal*
dihydrofarnesal**
7-hydroxy-3,7-dimethyl-octanal*
2,6-dimethyl-oct-5-enal
2-[4-(1-methylethyl)phenyl]-ethanal*
3-(3-isopropyl-phenyl)-butanal**
2-(3,7-dimethyoct-6-enoxy)-ethanal
1-carboxaldehyde-4-(4-methyl-3-pentenyl)-cyclohex-3-ene*
2,3,5,5,-tetramethyl-hexanal
longifolic aldehyde
2-methyl-4-(2,6,6-trimethylcyclohex-2-en-1-yl)-butanal*
2-methyl-3-(4-tert-butylphenyl)-propanal**
4-(1,1-dimethyl-ethyl)-benzene-propanal*
2-[4-(1-methyl-ethyl)-phenyl]-propanal
alpha-methyl-1,3-benzodioxole-5-propanal*
3,7-dimethyl-oct-6-enal*
2-methyl-3-(4-isopropylphenyl)-propionaldehyde*
4-(4-hydroxy-4-methyl-pentyl)-cyclohex-3-en-1-carboxaldehyde**
alpha-methyl-1,3-benzodioxole-5-propanal*
1-carboxaldehyde-4-(1,1-dimethylethyl)-cyclohexane
4-(octahydro-4,7-methano-5H-inden-5-ylidene)-butanal
[(3,7-dimethyl-6-octenyl)-oxy]-acetaldehyde**
whereby * indicates the preferred aldehydes and ** indicate the more preferred aldehydes.

Examples of ketones R¹O:
2-heptyl-cyclopentanone
2,2,6,10-tetrametyltricyclo-[5.4.0.0(6,10)]-undecan-4-one benzylacetone*
carvone*
1,2,3,5,6,7-hexahydro-1,1,2,3,3,-pentamentyl-4H-inden-4-one*
methyl heptenone*
dimethyl octenone*
2-(butan-2-yl)-cyclohexanone*
2-hexyl-cyclopent-2-en-1-one*
2-(1-methylethyl)-5-methyl-cyclohexanone*
2-(2-methylethyl)-5-methyl-cyclohexanone*
3-methyl-cyclopentadecanone
4-tert-pentyl-cyclohexanone*
3-oxo-2-pentyl-cyclopentane-acetic acid methyl ester**
1-(1,2,3,4,5,6,7,8-octahydro-2,3,8,8-tetramethyl-2-naphthalenyl)-ethanone*
3-methyl-5-propyl-cyclohex-2-en-1-one*
whereby * indicates the preferred ketones and ** indicate the more preferred ketone.

Examples of fluorescent whitening coumarins of formula II generated upon cleavage and constituting the respective residue in the compounds of formula I are:
7-(3-methyl-1H-pyrazol-1-yl)-3-phenyl-2H-1-benzopyran-2-one
7-(4-methyl-5-phenyl-2H-1,2,3-triazol-2-yl)-3-phenyl-2H-1-benzopyran-2-one
7-(2H-naphtho[1,2-d]triazol-2-yl)-3-phenyl-2H-1-benzopyran-2-one
3-(1H-pyrazol-1-yl)-7-(2H-1,2,3-triazol-2-yl)-2H-1-benzopyran-2-one
7-(dimethylamino)-1-methyl-3-phenyl-2(1H)-quinolinone
7-(diethylamino)-1-ethyl-3-phenyl-2(1H)-quinolinone
7-amino-4-methyl-2H-1-benzopyran-2-one
7-(dimethylamino)-4-methyl-2H-1-benzopyran-2-one
7-(diethylamino)-4-methyl-2H-1-benzopyran-2-one
7-hydroxy-4-methyl-2H-1-benzopyran-2-one
6,7-dihydroxy-2H-1-benzopyran-2-one

Examples for coumarins of formula II with olfactory properties generated upon cleavage and constituting the respective residue in the compounds of formula I are:
2H-1-benzopyran
3-methyl-benzopyran-2-one
8-(1,1-dimethylethyl)-6-methyl-benzopyrone
4-methyl-7-ethoxy-coumarin
6-methyl-2H-1-benzopyran

It is a matter of course, that it is not possible to give a complete list of the active coumarins, alcohols, aldehydes and ketones which are generated as a result of the desired cleavage of the acrylic acid esters of formula I by UV-light and/or by elevated temperatures. The skilled person is, however, quite aware of those alcohols, aldehydes, ketones and coumarins which provide the desired organoleptic, e.g. fragrance, flavour and odour masking, antimicrobial and/or brightening effects.

The compounds of formula I may preferably be used as sustained release odorants and flavours but also as sustained agents to mask or attenuate undesirable odours or to provide additional odours not initially present in consumer products, i.e. laundry detergents, fabric softeners, fabric softener sheets, toiletries and cosmetics such as sunscreens. Further applications are sustained brighteners and antimicrobial agents in the same products. The brighteners are especially useful for wool, rayon and polyamides. These compounds are also useful for flavouring and aromatizing tobacco products, e.g. cigarettes.

The amount required to produce the desired, overall effect varies depending upon the particular compounds of formula I chosen, the product in which it will be used, and the particular effect desired.

For example, depending upon the selection and concentration of the compound chosen, when a compound of the formula I is added either singly or as a mixture, e.g. to a laundry product composition at levels ranging from about 0.001 to about 10 % by weight, a coumarin and if desired an odoriferous alcohol or aldehyde or ketone in an organoleptically effective amount is released when the product is used. These newly formed odorant(s) serve to enhance the odour of the fragrance. Depending on the compound of formula I an antimicrobial agent or a brightener can be released.
Depending upon the selection and concentration, addition of the compounds I, either singly or as a mixture, to cigarette tobacco at levels ranging from about 5 ppm to about 50'000 ppm tends to enhance the smoking flavour and/or mask undesirable smoking odours. An important property of these compounds I is that the flavourant or odorant is covalently bound as a non-volatile compound and the flavourant or odorant is released only when the tobacco product is ignited and burns.

Addition of the compounds of formula I either separately or as a mixture at levels suitably ranging from about 5 ppm to about 50'000 ppm by weight onto the media enclosing the tobacco serves to incorporate the odorant/flavourant in the side-stream smoke of the tobacco. Air borne flavourants and/or odorants are thus introduced. This newly formed odorant or flavourant serves to enhance or mask the smoking odours depending upon selection and use levels of the compounds I.

As is evident from the above compilation of alcohols, aldehydes, ketones and coumarins, a broad range of known odorants or flavours or mixtures can be generated from precursors of the invention. While manufacturing compositions the precursors of the invention may be used according to methods known to the perfumer, such as e.g. from W.A. Poucher, Perfumes, Cosmetics, Soaps, 2, 7th Edition, Chapman and Hall, London 1974. The fluorescent whitening agents may be added in the same manner.

The compounds of formula I can be prepared by using standard methods known to the skilled chemist. Convenient methods are outlined in the Examples.

### Example 1

### (E)-3-(2-Hydroxy-phenyl)-2-methyl-acrylic acid ethyl ester

To a solution of 75.0 g (carbethoxyethylidene)triphenyl phosphorane in 350 ml of toluene, 23.2 g of salicylaldehyde was dropped in at 20°C while cooling in an ice-bath. After stirring at room temperature for 90 min., the reaction mixture was diluted with toluene and washed to neutrality with water. The organic phase was dried, filtered and evaporated to dryness. The resulting yellow oil was purified by chromatography to yield 35.5 g of a colourless solid.
- NMR (CDCl₃): δ 7.82 (s, 1H), 7.30-6.85 (m, 4H), 6.6 (s, OH), 4.35-4.17 (q, 2H), 2.04 (s, 3H), 1.40-1.28 (t, 3H)

### Example 5

### (E)-3-(2-Hydroxy-phenyl)-2-methyl-acrylic acid 3,7 dimethyl-oct-6-enyl ester

A solution of 6.0 g of (E)-3-(2-hydroxy-phenyl)-2-methylacrylic acid, 5.3 g of citronellol and 1 g of p-toluenesulfonic acid in 150 ml of cyclohexane was refluxed for 6.5 hours using a water separator. Then the reaction mixture was cooled down, diluted with hexane and washed to neutrality with saturated sodium bicarbonate and water. The organic phase was dried, filtered and evaporated to dryness. The resulting yellow oil was purified by chromatography to yield 6.45 g of a colourless oil.
- NMR (CDCl₃): δ 7.80 (s, 1H), 7.38-6.83 (m, 4H), 6.4 (s, OH), 5.09 (t, 1H) 4.25 (t, 2H), 2.10-1.90 (m, 5H), 1.88-1.08 (m, 11H), 0.93 (d, 3H)

### Example 6

### (E)-3-(2-Hydroxy-phenyl)-2-methyl-acrylic acid phenethyl ester

According to the same procedure of Example 1, (E)-3-(2-hydroxy-phenyl)-2-methyl-acrylic acid phenethyl ester was prepared from 3-(2-hydroxy-phenyl)-2-methyl-acrylic acid, phenyl ethyl alcohol and p-toluenesulfonic acid.

The compounds of Example 1 yield upon cleavage organoleptic coumarin, Examples 5 and 6 organoleptic coumarins.

### Example 8

### (E)-3-(2-Hydroxy-phenyl)-acrylic acid dec-9-enyl ester

A mixture of 5.0 g 3-(2-hydroxy-phenyl)-acrylic acid ethyl ester, 6.1 g dec-9-en-1-ol and 1.0 g tetraisopropyl-orthotitanate was heated to 150°C removing the ethanol formed. After stirring for 2.5 hours at this temperature, the reaction mixture was cooled, diluted with ether and washed with brine. The organic phase was dried and evaporated to dryness. The resulting oil was Kugelrohr-distilled, crystallized and recrystallized to yield 1.69 g of colourless crystals.
- NMR (CDCl₃): δ 8.11-7.96 (d, 1H), 7.55-7.40 (d, 1H), 7.32-7.15 (m, 1H), 6.99-6.72 (m, 2H), 6.71-6.57 (d, 1H), 5.93-5.69 (m, 1H), 5.07-4.88 (m, 2H), 4.31-4.15 (t, 2H), 2.12-1.93 (m, 2H), 1.85-1.55 (m, 2H), 1.54-1.15 (m, 10H) ppm.

### (E)-3-(2-Hydroxy-phenyl)-acrylic acid 2-ethyl-4-(2,2,3-trimethyl-cyclopent-3-enyl)-but-2-enyl ester

According to the same procedure, 3-(2-hydroxy-phenyl)-acrylic acid 2-ethyl-4-(2,2,3-trimethyl-cyclopent-3-enyl)-but-2-enyl ester was prepared from 3-(2-hydroxy-phenyl)-acrylic acid ethyl ester, 2-ethyl-4(2,2,3-trimethyl cyclopentyl-3-en-1-yl)-but-2-en-1-ol and tetraisopropyl-o-titanate.

### (E)-3-(2-Hydroxy-5-methyl-phenyl)-acrylic acid 2-ethyl-4-(2,2,3-trimethyl-cyclopent-3-enyl)-but-2-enyl ester

According to the same procedure, 3-(2-hydroxy-5-methyl-phenyl)-acrylic acid 2-ethyl-4-(2,2,3-trimethyl-cyclopent-3-enyl)-but-2-enyl ester was prepared from 3-(2-hydroxy-5-methyl-phenyl)-acrylic acid ethyl ester, 2-ethyl-4(2,2,3-trimethyl cyclopentyl-3-en-1-yl)-but-2-en-1-ol and tetraisopropyl-o-titanate.

### (E)-3-(2-Hydroxy-5-methyl-phenyl)-acrylic acid 3-methyl-5-phenyl-pentyl ester

According to the same procedure, 3-(2-hydroxy-5-methyl-phenyl)-acrylic acid 3-methyl-5-phenyl-pentyl ester was prepared from 3-(2-hydroxy-5-methyl-phenyl)-acrylic acid ethyl ester, 3-methyl-5-phenyl-pentanol and tetraisopropyl-o-titanate.

### (E)-3-(4-Diethylamino-2-hydroxy-phenyl)-but-2-enoic acid dec-9-enyl ester

According to the same procedure, 3-(4-diethylamino-2-hydroxy-phenyl)-but-2-enoic acid dec-9-enyl ester was prepared from 3-(4-diethylamino-2-hydroxy-phenyl)-but-2-enoic acid ethyl ester, dec-9-en-1-ol and tetraisopropyl-o-titanate.

### (E)-3-(4-Diethylamino-2-hydroxy-phenyl)-but-2-enoic acid 3-methyl-5-phenyl-pentyl ester

According to the same procedure, 3-(4-diethylamino-2-hydroxy-phenyl)-but-2-enoic acid 3-methyl-5-phenyl-pentyl ester was prepared from 3-(4-diethylamino-2-hydroxy-phenyl)-but-2-enoic acid ethyl ester, 3-methyl-5-phenyl-pentanol and tetraisopropyl-o-titanate.

### Example 9

### 3-[2-(tert-Butyl-dimethyl-silanyloxy)-phenyl]-acrylic acid tert-butyl-dimethyl-silyl ester

To a solution of 5.0 g of (E)-3-(2-hydroxy-phenyl)-acrylic acid and 4.6 g of imidazole in 100 ml of DMF was added at room temperature a solution of 10.1 g of TBDMS-Cl. The reaction mixture was stirred overnight, poured onto 200 ml of cold water and extracted 3x with 150 ml of MTBE. The combined organic phases were dried (MgSO₄) and evaporated to dryness. The resulting oil was dried at 0.08 Torr/120-170°C to remove excess of TBDMS-Cl to yield 11.4 g of 3-[2-(tert-butyl-dimethyl-silanyloxy)-phenyl]-acrylic acid tert-butyl-dimethyl-silyl ester as a yellowish oil.
- NMR: (CDCl₃) δ 7.96 (d, 1H), 7.52 (dd, 1H), 7.23 (m, 1H), 6.94 (m, 1H), 6.82 (dd, 1H), 6.37 (d, 1H), 1.00 (s, 9H), 0.97 (s, 9H), 0.32 (s, 6H), 0.22 (s, 6H)

### Example 10

### 3-[2-(tert-Butyl-dimethyl-silanyloxy)-phenyl]-acryloyl chloride

To a 0°C cold solution of 6.3 g of 3-[2-(tert-Butyl-dimethyl-silanyloxy)-phenyl]-acrylic acid tert-butyl-dimethyl-silyl ester and 8 drops of DMF in 15 ml of CH₂Cl₂ was added dropwise 2.0 ml of oxalyl chloride. After complete addition the reaction mixture was allowed to warm to room temperature and stirring was continued for 60 h. The reaction mixture was filtered, evaporated to dryness and taken up in MTBE. The solution was cooled in the refrigerator overnight, filtered and evaporated to yield 4.4 g of 3-[2-(tert-Butyl-dimethyl-silanyloxy)-phenyl]-acryloyl chloride.
- NMR (CDCl₃): δ 8.28 (d, 1H), 7.55 (dd, 1H), 7.35 (m, 1H), 7.01 (m, 1H), 6.87 (dd, 1H), 6.63 (d, 1H), 1.04 (s, 9H), 0.26 (s, 6H)

### Example 11

### 3-(2-Hydroxy-phenyl)-acrylic acid 3-(3-isopropyl-phenyl)-but-1-enyl ester

To a suspension of 0.72 g of NaH (55% in oil), previously washed with hexane, in 22.5 ml of THF was added 1.67 ml of tert-butanol over a period of 12 min. The reaction mixture was stirred at room temperature for 1.25 h, was then cooled to -15°C and 2.85 g of 3-(3-isopropyl-phenyl)-butanal (Florhydral) was slowly added. Stirring was continued for 45 min. and then this cold enolate solution was added via Teflon canula to a -5°C cold solution of 4.44 g of 3-[2-(*tert*-butyl-dimethyl-silanyloxy)-phenyl]-acryloyl chloride in 7.5 ml of THF. After complete addition stirring at -5°C was continued for 1 h, the reaction mixture was quenched with 80 ml of water / 40 ml of brine and was extracted with MTBE. The combined organic phases were washed with water/brine 2:1, dried (MgSO₄) and evaporated to dryness to yield 6.65 g of crude 3-[2-(tert-Butyl-dimethyl-silanyloxy)-phenyl]-acrylic acid 3-(3-isopropyl-phenyl)-but-1-enyl ester as a yellowish, viscous oil.

To a 0°C cold solution of 6.2 g of this oil in 70 ml of THF was added slowly 13.7 ml of a 1M TBAF/THF solution. The reaction mixture was stirred at 0°C for 1.5 h, poured onto 200 ml of H₂O and extracted 3x with 150 ml of MTBE. The combined organic phases were washed with brine, dried (MgSO₄) and evaporated to dryness. The resulting oil was purified by chromatography to yield 2.64 g of 3-(2-Hydroxy-phenyl)-acrylic acid 3-(3-isopropyl-phenyl)-but-1-enyl ester in form of a yellowish oil.
- NMR (CDCl₃): δ 8.12 and 8.01 (d, 1H), 7.50-6.80 (m, 10H), 6.66 and 6.56 (d, 1H), 5.75 and 5.13 (m, 1H), 4.13 (m, CH), 3.53 (m, CH), 2.89 (m, CH), 1.42 (d, 3H), 1.25 (d, 6H)

### Example 13

### (E)-3-(2-Hydroxy-phenyl)-acrylic acid 1-ethoxy-3-(3-isopropyl-phenyl)-butyl ester

A solution of 6.5 g of (E)-3-[2-(tert-Butyl-dimethyl-silanoxy)-phenyl]-acrylic acid and 5.5 g of 1-(3-ethoxy-1-methyl-allyl)-3-isopropyl-benzene, accessible from 3-(3-isopropyl-phenyl)-butyraldehyde via a two step procedure according to P.D. Bartlett and A.A. Frimer, Heterocycles 11, 419-435, (1978), in 25 ml of toluene was heated to reflux and stirred for 16 h. After concentration, the reaction mixture was purified by flash chromatography to yield 7.5 g of the intermediate (E)-3-[2-(tert-Butyl-dimethyl-silanoxy)-phenyl]-acrylic acid 1-ethoxy-3-(3-isopropyl-phenyl)-butyl ester as a pale yellow oil. This was dissolved in 50 ml THF and treated at 0°C with 4.7 g of TBAF•3H₂O. After 20 min., the mixture was concentrated and purification by flash chromatography yielded 2.2 g of the title ester (a mixture of diastereoisomers) as a pale yellow oil.
- NMR (CDCl₃): δ 8.12 and 8.08 (d, 1H), 7.47 (dt, 1H), 7.35 (br s, 1H), 7.31-7.18 (m, 2H), 7.12-6.98 (m, 3H), 6.92 (dt, 1H), 6.83 (dd, 1H), 6.68 and 6.61 (d, 1H), 5.96 and 5.77 (dd, 1H), 3.82-3.65 (m, 1H), 3.63-3.40 (m, 1H), 3.05-2.77 (m, 2H), 2.22-1.95 (m, 2H) and 1.38-1.12 (m, 12H)

### Example 14

### (E)-3-(2-Hydroxy-phenyl)-acrylic acid 3-(4-tert-butylphenyl)-1-ethoxy-propyl ester

A solution of 3.1 g of (E)-3-[2-(tert-Butyl-dimethyl-silanoxy)-phenyl]-acrylic acid, 3.0 g of 1-tert-butyl-4-(3-ethoxy-2-methyl-allyl)-benzene, accessible from 3-(4-tert-butyl-phenyl)-2-methyl-propionaldehyde via a two step procedure according to P.D. Bartlett and A.A. Frimer, Heterocycles 11, 419-435, (1978), and 20 mg of *p*-TSA in 25 ml of toluene was stirred at 0 °C for 4 h and at room temperature for 14 h. The reaction mixture was partitioned between saturated sodium carbonate and hexane and the organic layer was washed with brine, dried over (Na₂SO₄) and evaporated to dryness to yield 5.3 g of the crude intermediate (E)-3-[2-(tert-Butyl-dimethyl-silanoxy)-phenyl]-acrylic acid 3-(4-tert-butyl-phenyl)-1-ethoxypropyl ester as a pale yellow oil. This was dissolved in 20 ml of THF and treated at 0 °C with 3.2 g of solid TBAF•3H₂O. After 30 min., the mixture was concentrated and purification by repeated flash chromatography yielded 1.2 g of the title ester (a mixture of diastereoisomers) as a pale yellow oil.
- NMR (CDCl₃): δ 8.17 and 8.13 (d, 1H), 7.50 (ddd, 1H), 7.36-7.18 (m, 4H), 7.11 (br dd, 2H), 6.98-6.83 (m, 2H), 6.73 and 6.69 (d, 1H), 5.94 (t, 1H), 3.92-3.70 (m, 1H), 3.71-3.54 (m, 1H), 3.05-2.81 (m, 1H), 2.52-2.34 (m, 1H), 2.30-2.12 (m, 1H) and 1.31 (s, 9H), 1.33-1.20 (m, 3H), 0.97 and 0.90 (t, 3H)

### Example 15

Test cloth was washed with detergent to which one or more of the precursors of Examples 1-8, 11, 13 and 14 had been added. The cloth was then line dried. The cloth dried in sunlight had a distinct fragrance note, as determined by a trained panel. In contrast, the cloth dried without sunlight was olfactively neutral.

### Example 16

Test cloth was washed with a detergent and then a fabric softener, containing one ore more of the precursors of Examples 1-8, 11, 13 and 14, was added to the rinse cycle. The cloth was then line dried. The cloth dried in sunlight had a distinct fragrance note, as determined by a trained panel. In contrast, the cloth dried without sunlight was olfactively neutral.

### Example 17

A 1% solution of one or more of the products of Examples 1-8, 11, 13 and 14 in ethanol was applied to cigarette papers to produce levels of 5-50'000 ppm of each flavourant. The paper was incorporated in cigarettes and, upon burning, released a fragrant odour.

### Example 18

A broad spectrum (UV-A and UV-B) oil/water sunscreen lotion was prepared with 0,5%.

| Part A | | |
|---|---|---|
| | | |
| Recipe:% | Compound | Chemical Name |
| 2 % | PARSOL MCX | Octyl methoxycinnamate |
| 3 % | PARSOL 1789 | 4-4-Butyl-4'methoxy-dibenzoyl methane |
| 12 % | Cétiol LC | Coco-caprylate/caprate |
| 4 % | Dermol 185 | Isostearyl neopentanoate |
| 0,25 % | Diethyleneglycol monostearate | PEG-2-stearate |
| 1 % | Cetylalcohol | Cetylalcohol |
| 0,25 % | MPOB/PPOB | Methyl-propylparabene |
| 0,1 % | EDTA BD | EDTA-sodium salt |
| 1 % | Amphisol DEA (Giv.) | Diethanolamine cetylphosphate |

| | | |
|---|---|---|
| Part B | | |
| | | |
| 20 % | Permulene TR-1 (+%) | Acrylate C10-C30 Alkylacrylate |
| 50,1 % | water deion | water deion |
| 5 % | Propyleneglycol | 1,2-Propanediol |
| 0,8 % | KOH (10%) | Potassium hydroxide |

| | | |
|---|---|---|
| Part A was heated in a reactor to 85°C. | | |

Part B was slowly added within 10 min., followed by addition of KOH and 0.5% of the product of Example 5. The emulsion was then cooled and degassed.

## Claims

1. Compounds of the formula I 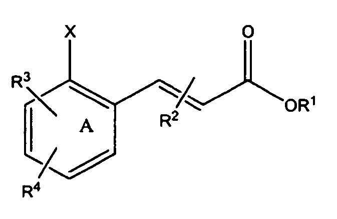 wherein
A is a benzene ring,
R¹ is a saturated or unsaturated, straight or branched, alicyclic or aromatic C₁₀-C₃₀ hydrocarbon residue, or
R¹ is a saturated or unsaturated, straight or branched, alicyclic or aromatic C₁₀-C₃₀ hydrocarbon residue containing one or more O and/or N atoms and/or C(O) groups and/or alkoxy groups, or
R¹ is a saturated or unsaturated, straight or branched, alicyclic or aromatic C₁₀-C₃₀ hydrocarbon residue substituted by an ionic substituent of the formula N(R⁵)₃⁺, in which R⁵ is the residue of an alkyl group with 1 to 30 carbon atoms;
R² in 2- or 3-position is methyl, or
R² in 2- or 3-position is a substituted heterocyclic residue of the formula 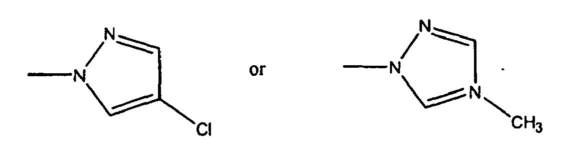
R³ and R⁴ stand for hydrogen, a straight or branched C₁-C₆ alkyl or C₁-C₆ alkoxy residue, a five membered heterocyclic residue containing N and/or O atoms, a five membered heterocyclic residue substituted by C₁-C₆ aliphatic and/or aromatic substituents, or -OH,
R², R³ and R⁴ may be the same or different,
X stands for -OH or NHR⁶, wherein R⁶ is hydrogen a saturated or unsaturated, straight or branched C₁-C₂₀ hydrocarbon or an aromatic or heterocyclic residue,
and the acrylic double bond is of the E configuration.

2. Compounds according to claim 1, wherein R¹ is the residue of an olfactory alcohol, of the formula R¹OH.

3. Compounds according to claim 1, wherein R¹ is the residue of the enol form of an olfactory aldehyde of formula R¹HO.

4. Compounds according to claim 1, wherein R¹ is the residue of the enol form of an olfactory ketone of formula R¹O.

5. Compounds according to claim 1, wherein R¹ is a C₁₀-C₃₀ hydrocarbon residue selected from alkyl, alkenyl arylalkyl residue carrying a 1-alkoxy, 1-aryloxy or 1-arylalkoxy residue.

6. Compounds according to one of the preceding claims, wherein the residue of formula Ia 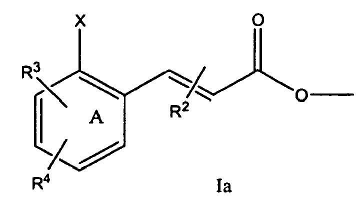 is the precursor for fragrant coumarins.

7. Compounds according to any one of the preceding claims, wherein R¹ is the residue of an olfactory alcohol, aldehyde or ketone and the residue of formula Ia 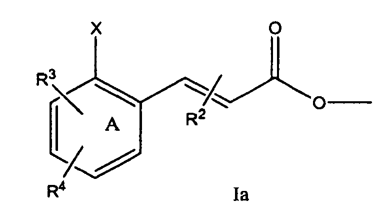 is the precursor for a fragrant coumarin.

8. Use of compounds of formula I 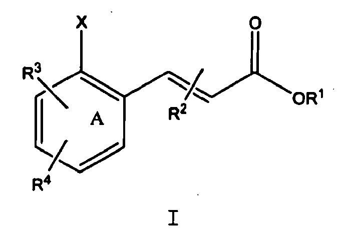 wherein
A is a benzene ring,
R¹ is a saturated or unsaturated, straight or branched, alicyclic or aromatic C₁₀-C₃₀ hydrocarbon residue, or
R¹ is a saturated or unsaturated, straight or branched, alicyclic or aromatic C₁₀-C₃₀ hydrocarbon residue containing one or more O and/or N atoms and/or C(O) groups and/or alkoxy groups, or
R¹ is a saturated or unsaturated, straight or branched, alicyclic or aromatic C₁₀-C₃₀ hydrocarbon residue substituted by an ionic substituent of the formula NR⁵₃⁺, in which R⁵ is the residue of an alkyl group with 1 to 30 carbon atoms;
R² in 2- or 3-position is methyl, or
R² in 2- or 3-position is a substituted heterocyclic residue of the formula 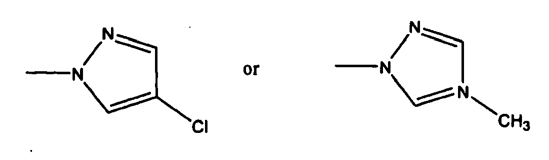
R³ and R⁴ stand for hydrogen, a straight or branched C₁-C₆ alkyl or C₁-C₆ alkoxy residue, a five membered heterocyclic residue containing N and/or O atoms, a five membered heterocyclic residue substituted by C₁-C₆ aliphatic and/or aromatic substituents, or -OH,
R², R³ and R⁴ may be the same or different,
X stands for -OH or NHR⁶, wherein R⁶ is hydrogen a saturated or unsaturated, straight or branched C₁-C₂₀ hydrocarbon or an aromatic or heterocyclic residue,
and the acrylic double bond is of the E configuration,
including such compounds of formula I wherein R² is hydrogen and such compounds wherein R¹ is the residue is the residue of an alcohol R¹OH selected from the group consisting of amyl alcohol, hexyl alcohol, 2-hexyl alcohol, heptyl alcohol, octyl alcohol, nonyl alcohol, 3-methyl-but-2-en-1-ol, 3-methyl-1-pentanol, cis-3-hexenol, cis-4-hexenol, 3,5,5-trimethyl-hexanol, oct-1-en-3-ol, cis-6-nonenol, 2,6-nonadien-1-ol, benzylalcohol, 1-phenyl-ethanol, 2-phenyl-ethanol, 2-phenyl-propanol, 3-phenyl-propanol, 2-(2-methylphenyl)-ethanol, 2-phenoxy-ethanol, 2-methoxy-4-methyl-phenol, 4-methyl-phenol, anisic alcohol, p-tolyl alcohol, cinnamic alcohol, vanillin, ethyl vanillin, 4-isopropyl-cyclohexanol, 3,3,5-trimethyl-cyclohexanol, 2-cyclohexyl-propanol and 2,6-dimethyl-heptan-2-ol; or
R¹ is the residue of the enol form of an aldehyde R¹HO selected from the group consisting of 2,4-dimethyl-cyclohex-3-ene-1-carboxaldehyde, 1-carboxaldehyde-2,4-dimethyl-cyclohex-3-ene, 3,5,5-trimethyl-hexanal, heptanal, octanal, nonanal, 3,5,5-trimethylhexanal, 2-phenyl-propanal, 4-methyl-phenyl-acetaldehyde and
2,3,5,5,-tetramethyl-hexanal;
as precursors for generating coumarins of formula II 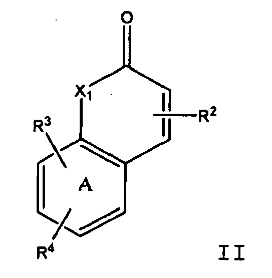 and a second compound selected from R¹OH, R¹HO and R¹O, wherein X₁ is O or NR⁶; and
A and R¹ to R⁶ have the same meaning as given for the compounds of formula I.

9. Use according to claim 8 in laundry products.

10. Use according to claim 8 in tobacco products.

11. Use according to claim 8 in cosmetics and toiletries.

12. A compound selected from (E)-3-(2-Hydroxy-phenyl)-2-methyl-acrylic acid 3,7 dimethyl-oct-6-enyl ester, (E)-3-(2-Hydroxy-phenyl)-2-methyl-acrylic acid phenethyl ester, (E)-3-(2-Hydroxy-phenyl)-acrylic acid dec-9-enyl ester, (E)-3-(2-Hydroxy-phenyl)-acrylic acid 2-ethyl-4-(2,2,3-trimethyl-cyclopent-3-enyl)-but-2-enyl ester, (E)-3-(2-Hydroxy-5-methyl-phenyl)-acrylic acid 2-ethyl-4-(2,2,3-trimethyl-cyclopent-3-enyl)-but-2-enyl ester, (E)-3-(2-Hydroxy-5-methyl-phenyl)-acrylic acid 3-methyl-5-phenylpentyl ester, (E)-3-(4-Diethylamino-2-hydroxy-phenyl)-but-2-enoic acid dec-9-enyl ester, (E)-3-(4-Diethylamino-2-hydroxy-phenyl)-but-2-enoic acid 3-methyl-5-phenyl-pentyl ester, 3-(2-Hydroxy-phenyl)-acrylic acid 3-(3-isopropylphenyl)-but-1-enyl ester, (E)-3-(2-Hydroxy-phenyl)-acrylic acid 1-ethoxy-3-(3-isopropyl-phenyl)-butyl ester and (E)-3-(2-Hydroxy-phenyl)-acrylic acid 3-(4-tert-butyl-phenyl)-1-ethoxy-propyl ester.

13. A method for generating coumarins of formula II 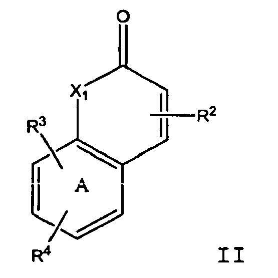 and an olfactory compound selected from R¹OH, R¹HO and R¹O, wherein X₁, A and R¹ to R⁴ have the same meaning as given in claim 8,**characterized in that** a compound of formula I as defined by claim 8 is exposed to UV-Light or elevated temperature.

## Patentansprüche

1. Verbindungen der Formel I 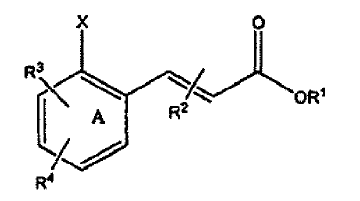 worin
A für einen Benzolring steht,
R¹ für einen gesättigten oder ungesättigten, geraden oder verzweigten, alicyclischen oder aromatischen C₁₀-C₃₀-Kohlenwasserstoffrest steht oder
R¹ für einen gesättigten oder ungesättigten, geraden oder verzweigten, alicyclischen oder aromatischen C₁₀-C₃₀-Kohlenwasserstoffrest mit einem oder mehreren 0- und/oder N-Atomen und/oder C(O)-Gruppen und/oder Alkoxygruppen steht oder
R¹ für einen gesättigten oder ungesättigten, geraden oder verzweigten, alicyclischen oder aromatischen C₁₀-C₃₀-Kohlenwasserstoffrest, der durch einen ionischen Substituenten der Formel N(R⁵)₃⁺, worin R⁵ für den Rest einer Alkylgruppe mit 1 bis 30 Kohlenstoffatomen steht, substituiert ist, steht;
R² in 2- oder 3-Position für Methyl steht oder
R² in 2- oder 3-Position für einen substituierten heterocyclischen Rest der Formel 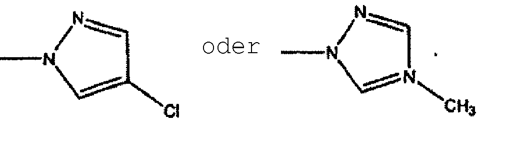 steht;
R³ und R⁴ für Wasserstoff, eine geraden oder verzweigten C₁-C₆-Alkyl- oder C₁-C₆-Alkoxyrest, einen fünfgliedrigen heterocyclischen Rest mit N- und/oder O-Atomen, einen fünfgliedrigen heterocyclischen Rest, der durch C₁-C₆-aliphatische und/oder aromatische Substituenten substituiert ist, oder -OH stehen,
R², R³ und R⁴ gleich oder verschieden sein können,
X für -OH oder NHR⁶, worin R⁶ Wasserstoff, einen gesättigten oder ungesättigten, geraden oder verzweigten C₁-C₂₀-Kohlenwasserstoffrest oder einen aromatischen oder heterocyclischen Rest bedeutet, steht,
und die acrylische Doppelbindung E-Konfiguration aufweist.

2. Verbindungen nach Anspruch 1, worin R¹ für den Rest eines olfaktorischen Alkohols der Formel R¹OH steht.

3. Verbindungen nach Anspruch 1, worin R¹ für den Rest der Enolform eines olfaktorischen Aldehyds der Formel R¹HO steht.

4. Verbindungen nach Anspruch 1, worin R¹ für den Rest der Enolform eines olfaktorischen Ketons der Formel R¹O steht.

5. Verbindungen nach Anspruch 1, worin R¹ für einen C₁₀-C₃₀-Kohlenwasserstoffrest steht, der unter einem Alkyl-, Alkenyl- oder Arylalkylrest mit einem 1-Alkoxy-, 1-Aryloxy- oder 1-Arylalkoxyrest ausgewählt ist.

6. Verbindungen nach einem der vorhergehenden Ansprüche, worin der Rest der Formel Ia 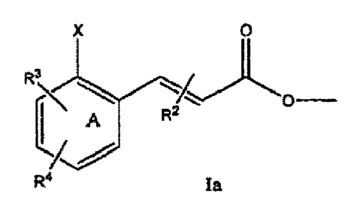 der Vorläufer für Duft-Cumarine ist.

7. Verbindungen nach einem der vorhergehenden Ansprüche, worin R¹ für den Rest eines olfaktorischen Alkohols, Aldehyds oder Ketons steht und der Rest der Formel Ia 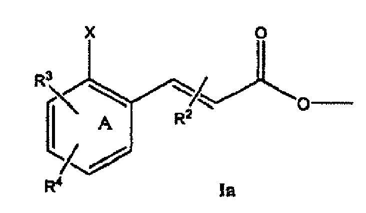 der Vorläufer für ein Duft-Cumarin ist.

8. Verwendung von Verbindungen der Formel I 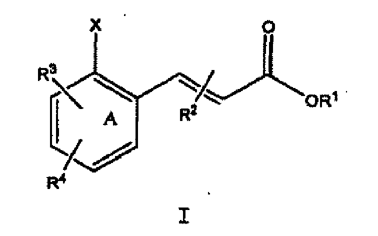 worin
A für einen Benzolring steht,
R¹ für einen gesättigten oder ungesättigten, geraden oder verzweigten, alicyclischen oder aromatischen C₁₀-C₃₀-Kohlenwasserstoffrest steht oder
R¹ für einen gesättigten oder ungesättigten, geraden oder verzweigten, alicyclischen oder aromatischen C₁₀-C₃₀-Kohlenwasserstoffrest mit einem oder mehreren 0- und/oder N-Atomen und/oder C(O)-Gruppen und/oder Alkoxygruppen steht oder
R¹ für einen gesättigten oder ungesättigten, geraden oder verzweigten, alicyclischen oder aromatischen C₁₀-C₃₀-Kohlenwasserstoffrest, der durch einen ionischen Substituenten der Formel NR⁵₃⁺, worin R⁵ für den Rest einer Alkylgruppe mit 1 bis 30 Kohlenstoffatomen steht, substituiert ist, steht;
R² in 2- oder 3-Position für Methyl steht oder
R² in 2- oder 3-Position für einen substituierten heterocyclischen Rest der Formel 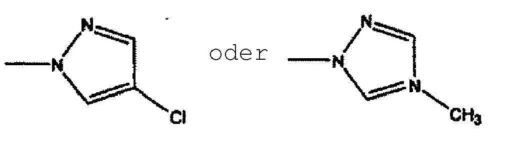 steht;
R³ und R⁴ für Wasserstoff, eine geraden oder verzweigten C₁-C₆-Alkyl- oder C₁-C₆-Alkoxyrest, einen fünfgliedrigen heterocyclischen Rest mit N- und/oder O-Atomen, einen fünfgliedrigen heterocyclischen Rest, der durch C₁-C₆-aliphatische und/oder aromatische Substituenten substituiert ist, oder -OH stehen,
R², R³ und R⁴ gleich oder verschieden sein können,
X für -OH oder NHR⁶, worin R⁶ Wasserstoff, einen gesättigten oder ungesättigten, geraden oder verzweigten C₁-C₂₀-Kohlenwasserstoffrest oder einen aromatischen oder heterocyclischen Rest bedeutet, steht,
und die acrylische Doppelbindung E-Konfiguration aufweist,
einschließlich solcher Verbindungen der Formel I, worin R² für Wasserstoff steht, und solcher Verbindungen der Formel I, worin R¹ für den Rest eines Alkohols R¹OH aus der Gruppe bestehend aus Amylalkohol, Hexylalkohol, 2-Hexylalkohol, Heptylalkohol, Octylalkohol, Nonylalkohol, 3-Methylbut-2-en-1-ol, 3-Methyl-1-pentanol, cis-3-Hexenol, cis-4-Hexenol, 3,5,5-Trimethylhexanol, Oct-1-en-3-ol, cis-6-Nonenol, 2,6-Nonadien-1-ol, Benzylalkohol, 1-Phenylethanol, 2-Phenylethanol, 2-Phenylpropanol, 3-Phenylpropanol, 2-(2-Methyl-phenyl)ethanol, 2-Phenoxyethanol, 2-Methoxy-4-methylphenol, 4-Methylphenol, Anisalkohol, p-Tolylalkohol, Zimtalkohol, Vanillin, Ethylvanillin, 4-Isopropylcyclohexanol, 3,3,5-Trimethylcyclohexanol, 2-Cyclohexylpropanol und 2,6-Dimethylheptan-2-ol steht oder
R¹ für den Rest der Enolform eines Aldehyds R¹HO aus der Gruppe bestehend aus 2,4-Dimethylcyclohex-3-en-1-carboxaldehyd, 1-Carboxaldehyd-2,4-dimethylcyclohex-3-en, 3,5,5-Trimethylhexanal, Heptanal, Octanal, Nonanal, 3,5,5-Trimethylhexanal, 2-Phenylpropanal, 4-Methylphenylacetaldehyd und 2,3,5,5-Tetramethylhexanal steht;
als Vorläufer zur Bildung von Cumarinen der Formel II 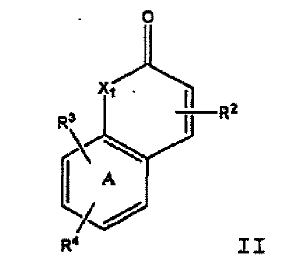 und einer unter R¹OH, R¹HO und R¹O ausgewählten zweiten Verbindung, worin X₁ für 0 oder NR⁶ steht und
A und R¹ bis R⁶ die gleiche Bedeutung wie oben für die Verbindungen der Formel I angegeben besitzen.

9. Verwendung nach Anspruch 8 in Waschmittelprodukten.

10. Verwendung nach Anspruch 8 in Tabakprodukten.

11. Verwendung nach Anspruch 8 in Kosmetika und Toilettenartikeln.

12. Verbindung, ausgewählt unter (E)-3-(2-Hydroxy-phenyl)-2-methylacrylsäure-3,7-dimethyloct-6-enylester, (E)-3-(2-Hydroxyphenyl)-2-methylacrylsäurephenethylester, (E)-3-(2-Hydroxyphenyl)acrylsäuredec-9-enylester, (E)-3-(2-Hydroxyphenyl)acrylsäure-2-ethyl-4-(2,2,3-trimethylcyclopent-3-enyl)but-2-enylester, (E)-3-(2-Hydroxy-5-methylphenyl)acrylsäure-2-ethyl-4-(2,2,3-trimethylcyclopent-3-enyl)but-2-enylester, (E)-3-(2-Hydroxy-5-methylphenyl)acrylsäure-3-methyl-5-phenylpentylester, (E)-3-(4-Diethylamino-2-hydroxyphenyl)but-2-ensäuredec-9-enylester, (E)-3-(4-Diethylamino-2-hydroxyphenyl)but-2-ensäure-3-methyl-5-phenylpentylester, 3-(2-Hydroxyphenyl)acrylsäure-3-(3-isopropylphenyl)but-1-enylester, (E)-3-(2-Hydroxyphenyl)acrylsäure-1-ethoxy-3-(3-isopropylphenyl)butylester und (E)-3-(2-Hydroxyphenyl)acrylsäure-3-(4-tert.-butylphenyl)-1-ethoxypropylester.

13. Verfahren zur Bildung von Cumarinen der Formel II 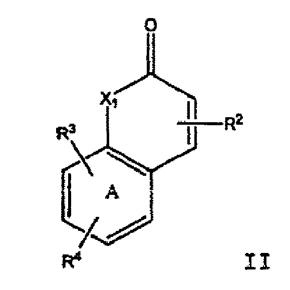 und einer unter R¹OH, R¹HO und R¹O ausgewählten olfaktorischen Verbindung, worin X₁, A und R¹ bis R⁴ die gleiche Bedeutung wie in Anspruch 8 angegeben besitzen, **dadurch gekennzeichnet, daß** man eine Verbindung der Formel I gemäß Anspruch 8 UV-Licht oder erhöhter Temperatur aussetzt.

## Revendications

1. Composés de formule I 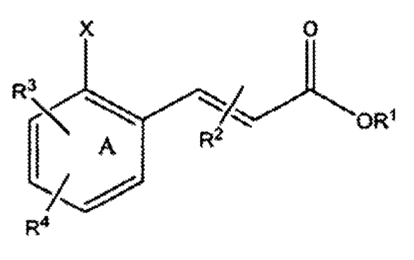 dans laquelle
A est un cycle benzène,
R¹ est un résidu hydrocarboné en C₁₀-C₃₀ saturé ou insaturé, linéaire ou ramifié, alicyclique ou aromatique, ou
R¹ est un résidu hydrocarboné en C₁₀-C₃₀ saturé ou insaturé, linéaire ou ramifié, alicyclique ou aromatique, contenant un ou plusieurs atomes 0 et/ou N et/ou groupes C(O) et/ou groupes alcoxy, ou
R¹ est un résidu hydrocarboné en C₁₀-C₃₀ saturé ou insaturé, linéaire ou ramifié, alicyclique ou aromatique, substitué par un substituant ionique de formule N(R⁵)₃⁺, dans laquelle R⁵ est le résidu d'un groupe alkyle contenant 1 à 30 atomes de carbone ;
R² en position 2 ou 3 est un méthyle, ou
R² en position 2 ou 3 est un résidu hétérocyclique substitué de formule 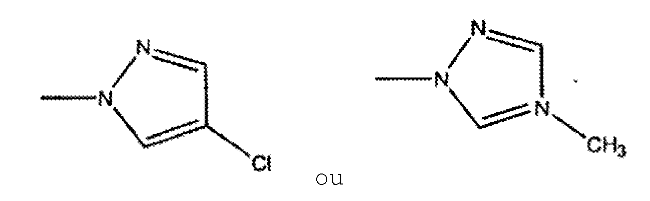
R³ et R⁴ représentent l'hydrogène, un résidu alcoxy en C₁-C₆ ou alkyle en C₁-C₆ linéaire ou ramifié, un résidu hétérocyclique à cinq éléments contenant des atomes N et/ou 0, un résidu hétérocyclique à cinq éléments substitué par des substituants aromatiques et/ou aliphatiques en C₁-C₆, ou -OH,
R² R³ et R⁴ peuvent être identiques ou différents,
X représente -OH ou NHR⁶, R⁶ étant l'hydrogène, un hydrocarbure en C₁-C₂₀ saturé ou insaturé, linéaire ou ramifié, ou un résidu aromatique ou hétérocyclique,
et la double liaison acrylique est de configuration E.

2. Composés selon la revendication 1, dans lesquels R¹ est le résidu d'un alcool olfactif, de formule R¹OH.

3. Composés selon la revendication 1, dans lesquels R¹ est le résidu de la forme énol d'un aldéhyde olfactif de formule R¹HO.

4. Composés selon la revendication 1, dans lesquels R¹ est le résidu de la forme énol d'une cétone olfactive de formule R¹O.

5. Composés selon la revendication 1, dans lesquels R¹ est un résidu hydrocarboné en C₁₀-C₃₀ choisi parmi un résidu alkyle, alcényle, arylalkyle portant un résidu 1-alcoxy, 1-aryloxy ou 1-arylalcoxy.

6. Composés selon l'une quelconque des revendications précédentes, dans lesquels le résidu de formule Ia 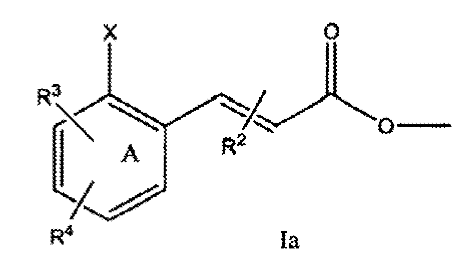 est le précurseur de coumarines fragrantes.

7. Composés selon l'une quelconque des revendications précédentes, dans lesquels R¹ est le résidu d'un alcool, aldéhyde ou cétone olfactif, et le résidu de formule Ia 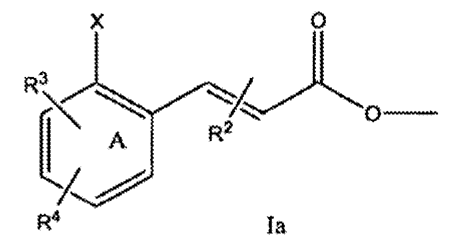 est le précurseur d'une coumarine fragrante.

8. Utilisation de composés de formule I 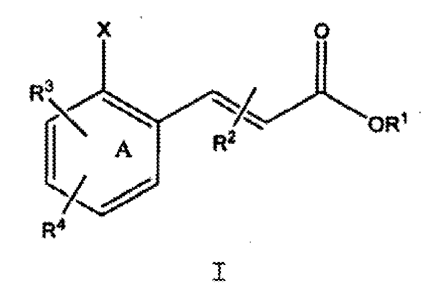 dans laquelle
A est un cycle benzène,
R¹ est un résidu hydrocarboné en C₁₀-C₃₀ saturé ou insaturé, linéaire ou ramifié, alicyclique ou aromatique, ou
R¹ est un résidu hydrocarboné en C₁₀-C₃₀ saturé ou insaturé, linéaire ou ramifié, alicyclique ou aromatique, contenant un ou plusieurs atomes 0 et/ou N et/ou groupes C(O) et/ou groupes alcoxy, ou
R¹ est un résidu hydrocarboné en C₁₀-C₃₀ saturé ou insaturé, linéaire ou ramifié, alicyclique ou aromatique, substitué par un substituant ionique de formule NR⁵₃⁺, dans laquelle R⁵ est le résidu d'un groupe alkyle contenant 1 à 30 atomes de carbone ;
R² en position 2 ou 3 est un méthyle, ou
R² en position 2 ou 3 est un résidu hétérocyclique substitué de formule 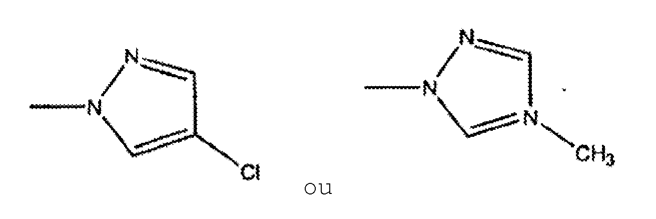
R³ et R⁴ représentent l'hydrogène, un résidu alcoxy en C₁-C₆ ou alkyle en C₁-C₆ linéaire ou ramifié, un résidu hétérocyclique à cinq éléments contenant des atomes N et/ou 0, un résidu hétérocyclique à cinq éléments substitué par des substituants aromatiques et/ou aliphatiques en C₁-C₆, ou -OH,
R², R³ et R⁴ peuvent être identiques ou différents,
X représente -OH ou NHR⁶, R⁶ étant l'hydrogène, un hydrocarbure en C₁-C₂₀ saturé ou insaturé, linéaire ou ramifié, ou un résidu aromatique ou hétérocyclique,
et la double liaison acrylique est de configuration E,
y compris les composés de formule I dans lesquels R² est l'hydrogène et les composés dans lesquels R¹ est le résidu d'un alcool R¹OH choisi dans le groupe constitué par l'alcool amylique, l'alcool hexylique, l'alcool 2-hexylique, l'alcool heptylique, l'alcool octylique, l'alcool nonylique, le 3-méthyl-but-2-én-1-ol, le 3-méthyl-1-pentanol, le cis-3-hexénol, le cis-4-hexénol, le 3,5,5-triméthyl-hexanol, l'oct-1-én-3-ol, le cis-6-nonénol, le 2,6-nonadién-1-ol, l'alcool benzylique, le 1-phényl-éthanol, le 2-phényl-éthanol, le 2-phényl-propanol, le 3-phényl-propanol, le 2-(2-méthylphényl)-éthanol, le 2-phénoxy-éthanol, le 2-méthoxy-4-méthyl-phénol, le 4-méthyl-phénol, l'alcool anisique, l'alcool p-tolylique, l'alcool cinnamique, la vanilline, l'éthylvanilline, le 4-isopropyl-cyclohexanol, le 3,3,5-triméthyl-cyclohexanol, le 2-cyclohexyl-propanol et le 2,6-diméthyl-heptan-2-ol ; ou
R¹ est le résidu de la forme énol d'un aldéhyde R¹HO choisi dans le groupe constitué par le 2,4-diméthyl-cyclohex-3-ène-1-carboxaldéhyde, le 1-carboxaldéhyde-2,4-diméthyl-cyclohex-3-ène, le 3,5,5-triméthyl-hexanal, l'heptanal, l'octanal, le nonanal, le 3,5,5-triméthylhexanal, le 2-phényl-propanal, le 4-méthyl-phényl-acétaldéhyde et le 2,3,5,5-tétraméthyl-hexanal ;
en tant que précurseurs pour la génération de coumarines de formule II 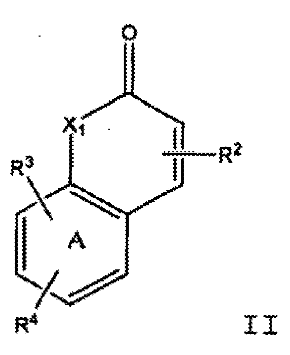 et d'un second composé choisi parmi R¹OH, R¹HO et R¹O,
X₁ représentant 0 ou NR⁶ ; et
A et R¹ à R⁶ ayant la même signification que celle donnée pour les composés de formule I.

9. Utilisation selon la revendication 8, dans des produits de lessive.

10. Utilisation selon la revendication 8, dans des produits de tabac.

11. Utilisation selon la revendication 8, dans des produits cosmétiques et de toilette.

12. Composé choisi parmi l'ester 3,7-diméthyl-oct-6-énylique de l'acide (E)-3-(2-hydroxy-phényl)-2-méthyl-acrylique, l'ester phénéthylique de l'acide (E)-3-(2-hydroxy-phényl)-2-méthyl-acrylique, l'ester déc-9-énylique de l'acide (E)-3-(2-hydroxy-phényl)-acrylique, l'ester 2-éthyl-4-(2,2,3-triméthyl-cyclopent-3-ényl)-but-2-énylique de l'acide (E)-3-(2-hydroxy-phényl)-acrylique, l'ester 2-éthyl-4-(2,2,3-triméthyl-cyclopent-3-ényl)-but-2-énylique de l'acide (E)-3-(2-hydroxy-5-méthyl-phényl)-acrylique, l'ester 3-méthyl-5-phényl-pentylique de l'acide (E)-3-(2-hydroxy-5-méthyl-phényl)-acrylique, l'ester déc-9-énylique de l'acide (E)-3-(4-diéthylamino-2-hydroxy-phényl)-but-2-énoïque, l'ester 3-méthyl-5-phényl-pentylique de l'acide (E)-3-(4-diéthylamino-2-hydroxy-phényl)-but-2-énoïque, l'ester 3-(3-isopropyl-phényl)-but-1-énylique de l'acide 3-(2-hydroxy-phényl)-acrylique, l'ester 1-éthoxy-3-(3-isopropyl-phényl)-butylique de l'acide (E)-3-(2-hydroxy-phényl)-acrylique et l'ester 3-(4-tert-butyl-phényl)-1-éthoxy-propylique de l'acide (E)-3-(2-hydroxy-phényl)-acrylique.

13. Procédé de génération de coumarines de formule II 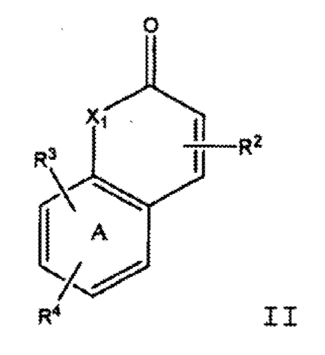 et d'un composé olfactif choisi parmi R¹OH, R¹HO et R¹O, X₁, A et R¹ à R⁴ ayant la même signification que celle donnée dans la revendication 8, **caractérisé en ce qu'**un composé de formule I tel que défini par la revendication 8 est exposé à de la lumière UV ou à une température élevée.
